# EUROPEAN PATENT APPLICATION

(11) **EP 4 467 156 A1**
(43) Date of publication of application: **27.11.2024**
(21) Application number: 23743342.0
(22) Date of filing: 20.01.2023
(51) Int. Cl.: A61K 45/00, A61K 31/4178, A61P 1/16, A61P 13/12, A61P 29/00, A61P 43/00

(54) **PHARMACEUTICAL COMPOSITION FOR SUPPRESSING ORGAN FIBROSIS**

(30) Priority: 21.01.2022 JP 2022007849
(71) Applicant: NAGASAKI UNIVERSITY, Nagasaki 852-8521 (JP)
(72) Inventor: LI Tao-Sheng, Nagasaki-shi, Nagasaki 852-8521 (JP); GU Weili, Nagasaki-shi, Nagasaki 852-8521 (JP)
(74) Representative: Witte, Weller & Partner Patentanwälte mbB
(86) International application number: PCT/JP2023/001676
(87) International publication number: WO 2023/140350

(57) **Abstract**

The present invention provides a pharmaceutical composition for inhibiting fibrosis in an organ, comprising an angiotensin II receptor antagonist as an active ingredient, wherein the composition is used so that the angiotensin II receptor antagonist is administered at a daily dose that is 0.2 times or less the daily dose of the angiotensin II receptor antagonist used as an antihypertensive.

## Description

### TECHNICAL FIELD

The present invention relates to a pharmaceutical composition for inhibiting fibrosis in organs.

### BACKGROUND ART

Fibrosis generally occurs in liver with chronic injury or inflammation. The key cellular mediator of liver fibrosis is well known as hepatic stellate cells (HSCs), which when activated serve as the primary collagen-producing cells. Therefore, a viable approach for reducing the activity of HSCs is arising for prevention and treatment of liver fibrosis.

Cells in our body are always perceiving the dynamic changes of various surrounding biomechanics, such as hydrostatic pressure generated by interstitial fluid and stiffness generated by extracellular matrix accumulation. The molecular process that cells convert physical cues into biological responses when subjected to biodynamic stimuli is collectively referred to as mechanotransduction, which is of fundamental importance to modify the biological properties of cells.

In diseased liver, the intrahepatic hydrostatic pressure is elevated due to the increased production of inflammatory cytokines and the enhanced vascular permeability. As the biological characteristics of HSCs can be largely affected by biomechanics in surrounding microenvironment, the elevation of hydrostatic pressure in diseased liver may modify the mechanotransduction properties of HSCs to regulate the development and progression of liver fibrosis. Therefore, the interference of mechanotransduction signaling to HSCs is considered as an effective therapeutic strategy on liver fibrosis.

Angiotensin II receptor blockers (ARBs), such as losartan, are known to protect against cardiac fibrosis by modulating mechanotransduction pathways, including Ras homolog family member A (RhoA) and its downstream effectors, Rho-associated protein kinase (ROCK) and myosin light chain (MLC) (non-patent literature 1 and 2). It has been reported that losartan effectively relieves acute lung injury caused by mechanical stimulation and mitigates liver fibrosis caused by non-alcoholic steatohepatitis in rats (non-patent literature 3 and 4). However, the effect of ARBs on organ fibrosis and the relevant mechanism largely remain unclear.

### CITATION LIST

### NON-PATENT LITERATURE

Non-patent literature 1: Ma Z-G, Yuan Y-P, Wu H-M, Zhang X, Tang Q-Z. Cardiac fibrosis: new insights into the pathogenesis. Int J Biol Sci 14: 1645-1657, 2018. doi: 10.7150/ijbs.28103.
Non-patent literature 2: Yamakawa T, Tanaka S, Numaguchi K, Yamakawa Y, Motley ED, Ichihara S, Inagami T. Involvement of Rho-kinase in angiotensin II-induced hypertrophy of rat vascular smooth muscle cells. Hypertens (Dallas, Tex 1979) 35: 313-318, 2000. doi: 10.1161/01.hyp.35.1.313.
Non-patent literature 3: Yao S, Feng D, Wu Q, Li K, Wang L. Losartan Attenuates Ventilator-Induced Lung Injury. J Surg Res 145: 25-32, 2008. doi: https://doi.org/10.1016/j.jss.2007.03.075.
Non-patent literature 4: Sawada Y, Kawaratani H, Kubo T, Fujinaga Y, Furukawa M, Saikawa S, Sato S, Seki K, Takaya H, Okura Y, Kaji K, Shimozato N, Mashitani T, Kitade M, Moriya K, Namisaki T, Akahane T, Mitoro A, Yamao J, Yoshiji H. Combining probiotics and an angiotensin-II type 1 receptor blocker has beneficial effects on hepatic fibrogenesis in a rat model of non-alcoholic steatohepatitis. Hepatol Res 49: 284-295, 2019. doi: https://doi.org/10.1111/hepr.13281.

### SUMMARY OF INVENTION

### TECHNICAL PROBLEM

An object of the invention is to provide a pharmaceutical composition for effectively inhibiting fibrosis in organs.

### SOLUTION TO PROBLEM

The present invention was made to solve the above problems and includes the following.
[1] A pharmaceutical composition for inhibiting fibrosis in an organ, comprising an angiotensin II receptor antagonist as an active ingredient, wherein the composition is used so that the angiotensin II receptor antagonist is administered at a daily dose that is 0.2 times or less the daily dose of the angiotensin II receptor antagonist used as an antihypertensive.
[2] The pharmaceutical composition according to the above [1], wherein the composition is used so that the angiotensin II receptor antagonist is administered at a daily dose that is 0.1 times or less the daily dose of the angiotensin II receptor antagonist used as an antihypertensive.
[3] The pharmaceutical composition according to the above [2], wherein the composition is used so that the angiotensin II receptor antagonist is administered at a daily dose that is 0.05 times or less the daily dose of the angiotensin II receptor antagonist used as an antihypertensive.
[4] The pharmaceutical composition according to any one of the above [1] to [3], wherein the angiotensin II receptor antagonist is a compound selected from the group consisting of losartan, candesartan, valsartan, telmisartan, olmesartan, irbesartan and azilsartan, or a pharmaceutically acceptable salt thereof.
[5] The pharmaceutical composition according to the above [4], wherein the angiotensin II receptor antagonist is losartan.
[6] The pharmaceutical composition according to any one of the above [1] to [5], wherein the organ is at least one selected from liver, kidney and heart.
[7] The pharmaceutical composition according to the above [6], wherein the organ is liver and/or kidney.
[8] The pharmaceutical composition according to any one of the above [1] to [7], wherein the fibrosis in the organ is fibrosis induced by a biomechanical change in the organ.
[9] The pharmaceutical composition according to the above [8], wherein the biomechanical change in the organ is a change in hydrostatic pressure.

### ADVANTAGEOUS EFFECTS OF INVENTION

The present invention provides a pharmaceutical composition for effectively inhibiting fibrosis in organs.

### BRIEF DESCRIPTION OF DRAWINGS

Fig. 1 shows the procedure for production of partial inferior vena cava (IVC) ligation model mice.
Fig. 2 shows the body weight and organ weights of mice that were subjected to partial IVC ligation surgery and then orally received low-dose losartan for 8 weeks. (A) shows the body weight, (B) shows liver/body weight ratios, and (C) shows spleen/body weight ratios.
Fig. 3 shows the levels of AST and ALT in serum collected from mice that were subjected to partial IVC ligation surgery and then orally received low-dose losartan for 8 weeks. (A) shows the levels of AST, (B) shows the levels of ALT, and (C) shows ALT/AST ratios.
Fig. 4 shows Masson's trichrome staining of liver tissues from mice that were subjected to partial IVC ligation surgery and then orally received low-dose losartan for 8 weeks. (A) shows representative images of staining in each group (scale bar is 500 µm), and (B) shows comparison of the percentages of positive area of Masson's trichrome staining of the groups by image analysis.
Fig. 5 shows apoptotic cells observed in liver tissues from mice that were subjected to partial IVC ligation surgery and then orally received low-dose losartan for 8 weeks. (A) shows representative images of TUNEL staining in each group, and (B) shows comparison of the percentages of positive area of TUNEL staining by image analysis.
Fig. 6 shows immunostaining of α-SMA in liver tissues from mice that were subjected to partial IVC ligation surgery and then orally received low-dose losartan for 8 weeks. (A) shows representative images of immunostaining of α-SMA in each group, and (B) shows comparison of signal intensity of α-SMA expression by image analysis.
Fig. 7 shows immunostaining of RhoA in liver tissues from mice that were subjected to partial IVC ligation surgery and then orally received low-dose losartan for 8 weeks. (A) shows representative images of immunostaining of RhoA in each group, and (B) shows comparison of signal intensity of RhoA expression by image analysis.
Fig. 8 shows immunostaining of ROCK1 in liver tissues from mice that were subjected to partial IVC ligation surgery and then orally received low-dose losartan for 8 weeks. (A) shows representative images of immunostaining of ROCK1 in each group, and (B) shows comparison of signal intensity of ROCK1 expression by image analysis.
Fig. 9 shows immunostaining of ROCK2 in liver tissues from mice that were subjected to partial IVC ligation surgery and then orally received low-dose losartan for 8 weeks. (A) shows representative images of immunostaining of ROCK2 in each group, and (B) shows comparison of signal intensity of ROCK2 expression by image analysis.
Fig. 10 shows immunostaining of p-MLC2 in liver tissues from mice that were subjected to partial IVC ligation surgery and then orally received low-dose losartan for 8 weeks. (A) shows representative images of immunostaining of p-MLC2 in each group, and (B) shows comparison of signal intensity of p-MLC2 expression by image analysis.
Fig. 11 shows the results of RT-qPCR of AT1R, ACTA2, TGFB1 and COL1A1 in ex vivo hydrostatic pressure loading test in human hepatic stellate cells (HSCs). (A) shows the results of AT1R, (B) shows the results of ACTA2, (C) shows the results of TGFB1, and (D) shows the results of COL1A1.
Fig. 12 shows immunohistochemistry of AT1R in ex vivo hydrostatic pressure loading test in HSCs. Representative images of immunostaining of AT1R in each group are shown.
Fig. 13 shows immunohistochemistry of AT1R in ex vivo hydrostatic pressure loading test in HSCs. (A) shows comparison of signal intensity of AT1R expression in the cytoplasm by image analysis, (B) shows comparison of signal intensity of AT1R expression in the nucleus by image analysis, and (C) shows comparison of cytoplasm signal intensity/nucleus signal intensity.
Fig. 14 shows immunohistochemistry of α-SMA in ex vivo hydrostatic pressure loading test in HSCs. (A) shows representative images of immunostaining of α-SMA in each group (scale bar is 30 µm), and (B) shows comparison of signal intensity of α-SMA expression by image analysis.
Fig. 15 shows immunohistochemistry of TGF-β1 in ex vivo hydrostatic pressure loading test in HSCs. (A) shows representative images of immunostaining of TGF-β1 in each group (scale bar is 30 µm), and (B) shows comparison of signal intensity of TGF-β1 expression by image analysis.
Fig. 16 shows immunohistochemistry of type 1 collagen in ex vivo hydrostatic pressure loading test in HSCs. (A) shows representative images of immunostaining of type 1 collagen in each group (scale bar is 30 µm), and (B) shows comparison of signal intensity of type 1 collagen expression by image analysis.
Fig. 17 shows the results of RT-qPCR of RhoA, ROCK1 and ROCK2 in ex vivo hydrostatic pressure loading test in HSCs. (A) shows the results of RhoA, (B) shows the results of ROCK 1, and (C) shows the results of ROCK2.
Fig. 18 shows immunohistochemistry of RhoA in ex vivo hydrostatic pressure loading test in HSCs. (A) shows representative images of immunostaining of RhoA in each group (scale bar is 30 µm), and (B) shows comparison of signal intensity of RhoA expression by image analysis.
Fig. 19 shows immunohistochemistry of ROCK1 in ex vivo hydrostatic pressure loading test in HSCs. (A) shows representative images of immunostaining of ROCK1 in each group (scale bar is 30 µm), and (B) shows comparison of signal intensity of ROCK1 expression by image analysis.
Fig. 20 shows immunohistochemistry of ROCK2 in ex vivo hydrostatic pressure loading test in HSCs. (A) shows representative images of immunostaining of ROCK2 in each group (scale bar is 30 µm), and (B) shows comparison of signal intensity of ROCK2 expression by image analysis.
Fig. 21 shows phalloidin staining for F-actin fibers and immunohistochemistry of p-MLC2 in ex vivo hydrostatic pressure loading test in HSCs. (A) shows representative images of staining in each group (scale bar is 30 µm), and (B) shows comparison of signal intensity of ROCK2 expression by image analysis.
Fig. 22 shows the procedure for production of unilateral ureteral obstruction (UUO)-induced renal fibrosis model mice.
Fig. 23 shows the body weight of mice measured prior to UUO surgery and 1 and 2 weeks post-surgery.
Fig. 24 shows a photograph showing the left and right kidneys of mice that orally received low- or high-dose losartan for two weeks after UUO surgery or mice that did not receive losartan after UUO surgery.
Fig. 25 shows the levels of BUN and CRE in serum collected from mice that orally received low- or high-dose losartan for two weeks after UUO surgery or mice that did not receive losartan after UUO surgery. (A) shows the levels of BUN and (B) shows the levels of CRE.

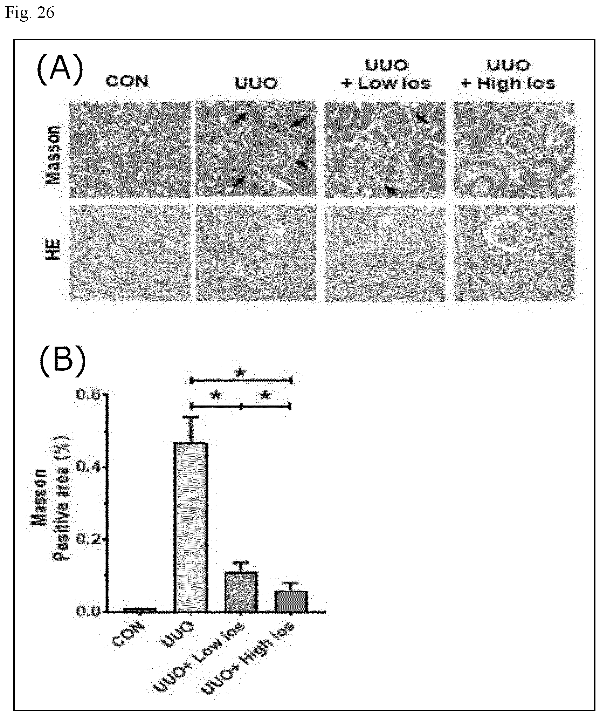

Fig. 26 shows Masson's trichrome staining and Hematoxylin and Eosin staining of kidney tissues from mice that orally received low- or high-dose losartan for two weeks after UUO surgery or mice that did not receive losartan after UUO surgery. (A) shows representative images of staining in each group. (B) shows comparison of the percentages of positive area of Masson's trichrome staining of the groups by image analysis.

### DESCRIPTION OF EMBODIMENTS

The present invention provides a pharmaceutical composition for inhibiting fibrosis in an organ, comprising an angiotensin II receptor antagonist as an active ingredient (hereinafter referred to as "the pharmaceutical composition of the invention"). The pharmaceutical composition of the invention is used so that the angiotensin II receptor antagonist is administered at a lower dose than the dose of the angiotensin II receptor antagonist used as an antihypertensive.

The daily dose of the pharmaceutical composition of the invention is not particularly limited as long as the daily dose of the angiotensin II receptor antagonist is lower than the daily dose of the angiotensin II receptor antagonist used as an antihypertensive. The daily dose of the pharmaceutical composition of the invention may be such that the daily dose of the angiotensin II receptor antagonist is 0.2 times or less, 0.1 times or less, 0.05 times or less, 0.04 times or less, 0.03 times or less, or 0.02 times or less the daily dose of the angiotensin II receptor antagonist used as an antihypertensive.

Angiotensin II receptor antagonists are also called "angiotensin receptor antagonists," "angiotensin receptor blockers," or "ARBs." Angiotensin II receptor antagonists are clinically widely used as therapeutic drugs for hypertension (antihypertensives). An angiotensin II receptor antagonist exerts its antihypertensive effect by binding to the angiotensin II type 1 receptor (AT1 receptor) and blocking the bonding between angiotensin II and the AT1 receptor.

The angiotensin II receptor antagonist used herein may be any compound that has the effect as described above, and may include, for example, losartan, candesartan, valsartan, telmisartan, olmesartan, irbesartan and azilsartan, and a pharmaceutically acceptable salt thereof. The angiotensin II receptor antagonist is preferably losartan or a pharmaceutically acceptable salt thereof. The pharmaceutical composition of the invention may contain two or more angiotensin II receptor antagonists.

The term "pharmaceutically acceptable salt" may refer to any salt that does not affect the activity of the angiotensin II receptor antagonist. Examples of the pharmaceutically acceptable salt include a salt with an inorganic acid, such as sulfuric acid, hydrochloric acid, hydrobromic acid, phosphoric acid, or nitric acid; a salt with an organic acid, such as acetic acid, oxalic acid, lactic acid, tartaric acid, fumaric acid, maleic acid, citric acid, benzenesulfonic acid, methanesulfonic acid, p-toluenesulfonic acid, benzoic acid, camphor sulfonic acid, ethanesulfonic acid, glucoheptonic acid, gluconic acid, glutamic acid, glycolic acid, malic acid, malonic acid, mandelic acid, galactaric acid, or naphthalene-2-sulfonic acid; a salt with one or more metal ions, such as lithium ion, sodium ion, potassium ion, calcium ion, magnesium ion, zinc ion, or aluminum ion; a salt with an amine, such as ammonia, arginine, lysine, piperazine, choline, diethylamine, 4-phenylcyclohexylamine, 2-aminoethanol, or benzathine; etc. The term "pharmaceutically acceptable salt" as used herein also includes a hydrate and a solvate. Examples of a solvent that forms a hydrate or a solvate include, but are not limited to, water, and physiologically acceptable organic solvent, such as ethanol or acetone.

Losartan is a compound with the structure shown below, and well-known as an active ingredient of therapeutic formulations for hypertension. Losartan potassium, which is a potassium salt of losartan, has been approved as an ethical drug in Japan, and registered in the National Health Insurance Price List. Losartan potassium and losartan potassium tablets are registered in the Japanese Pharmacopoeia.

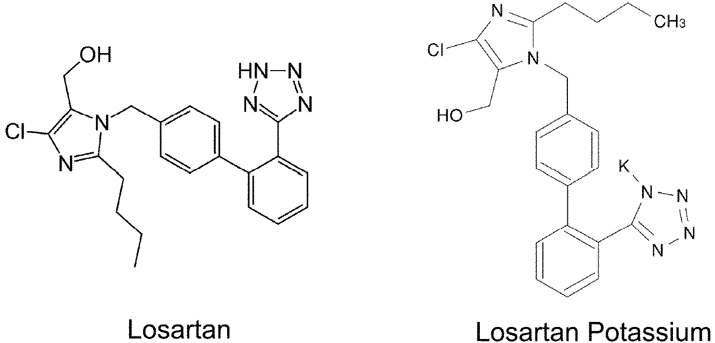

Candesartan is a compound with the structure shown below, and well-known as an active ingredient of therapeutic formulations for hypertension. Candesartan cilexetil (cilexetil ester of candesartan), which is a prodrug of candesartan, has been approved as an ethical drug in Japan, and registered in the National Health Insurance Price List. Candesartan cilexetil and candesartan cilexetil tablets are registered in the Japanese Pharmacopoeia. 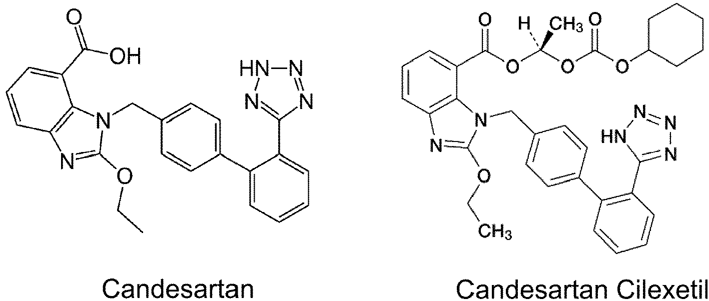 and enantiomer

Valsartan is a compound with the structure shown below, and well-known as an active ingredient of therapeutic formulations for hypertension. Valsartan has been approved as an ethical drug in Japan, and registered in the National Health Insurance Price List. Valsartan and valsartan tablets are registered in the Japanese Pharmacopoeia.

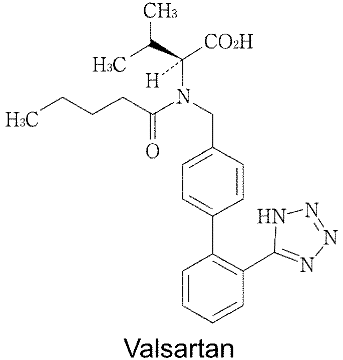

Telmisartan is a compound with the structure shown below, and well-known as an active ingredient of therapeutic formulations for hypertension. Telmisartan has been approved as an ethical drug in Japan, and registered in the National Health Insurance Price List. Telmisartan and telmisartan tablets are registered in the Japanese Pharmacopoeia.

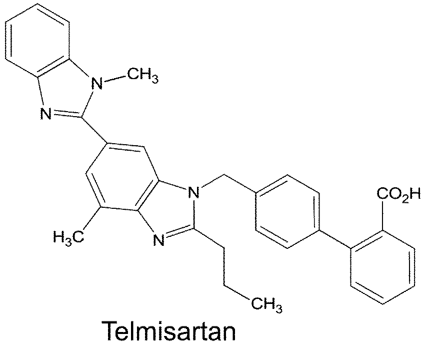

Olmesartan is a compound with the structure shown below, and well-known as an active ingredient of therapeutic formulations for hypertension. Olmesartan medoxomil (medoxomil ester of olmesartan), which is a prodrug of olmesartan, has been approved as an ethical drug in Japan, and registered in the National Health Insurance Price List. Olmesartan medoxomil and olmesartan medoxomil tablets are registered in the Japanese Pharmacopoeia.

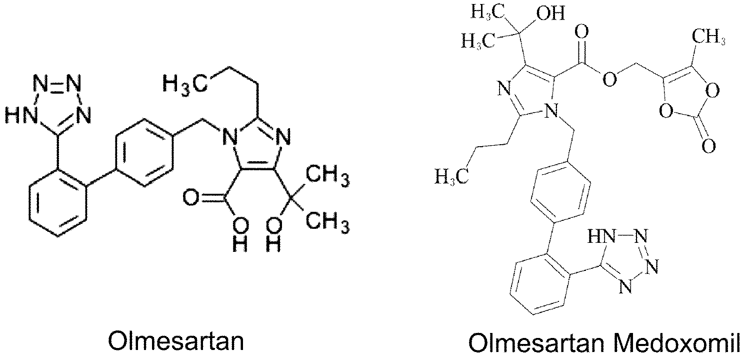

Irbesartan is a compound with the structure shown below, and well-known as an active ingredient of therapeutic formulations for hypertension. Irbesartan has been approved as an ethical drug in Japan, and registered in the National Health Insurance Price List. Irbesartan and irbesartan tablets are registered in the Japanese Pharmacopoeia.

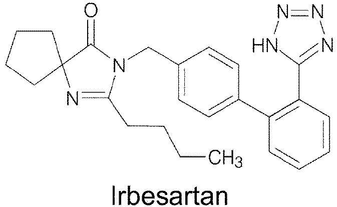

Azilsartan is a compound with the structure shown below, and well-known as an active ingredient of therapeutic formulations for hypertension. Azilsartan has been approved as an ethical drug in Japan, and registered in the National Health Insurance Price List. Azilsartan and azilsartan tablets are registered in the Japanese Pharmacopoeia.

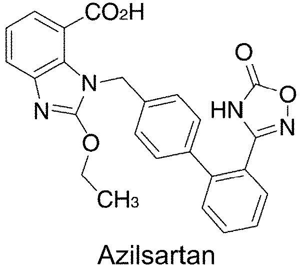

The daily dose of losartan potassium for a patient with hypertension is 25 mg to 100 mg (see the package insert of NU-LOTAN (registered trademark) tablets (Organon Co., Ltd.)). Accordingly, the daily dose of losartan or losartan potassium to be used as an active ingredient of the pharmaceutical composition of the invention for inhibiting fibrosis in organs may be 20 mg or less, 10 mg or less, 5 mg or less, 2.5 mg or less, 2.0 mg or less, 1.5 mg or less, 1.25 mg or less, 1.0 mg or less, 0.75 mg or less, or 0.5 mg or less. The lower limit of the daily dose is not limited to a particular amount as long as the inhibitory effect on fibrosis is exerted, and may be, for example, 0.1 mg or more, 0.2 mg or more, 0.3 mg or more, or 0.4 mg or more.

The daily dose of candesartan cilexetil for a patient with hypertension is 4 mg to 12 mg (see the package insert of BLOPRESS (registered trademark) tablets (Takeda Pharmaceutical Company Limited.)). Accordingly, the daily dose of candesartan or candesartan cilexetil to be used as an active ingredient of the pharmaceutical composition of the invention for inhibiting fibrosis in organs may be 2.4 mg or less, 1.2 mg or less, 1.0 mg or less, 0.8 mg or less, 0.6 mg or less, 0.4 mg or less, 0.2 mg or less, 0.1 mg or less, or 0.08 mg or less. The lower limit of the daily dose is not limited to a particular amount as long as the inhibitory effect on fibrosis is exerted, and may be, for example, 0.02 mg or more, 0.04 mg or more, 0.06 mg or more, or 0.07 mg or more.

The daily dose of valsartan for a patient with hypertension is 40 mg to 160 mg (see the package insert of Diovan (registered trademark) tablets (Novartis Pharma K.K.)). Accordingly, the daily dose of valsartan to be used as an active ingredient of the pharmaceutical composition of the invention for inhibiting fibrosis in organs may be 32 mg or less, 16 mg or less, 8 mg or less, 5 mg or less, 4 mg or less, 3 mg or less, 2 mg or less, 1 mg or less, or 0.8 mg or less. The lower limit of the daily dose is not limited to a particular amount as long as the inhibitory effect on fibrosis is exerted, and may be, for example, 0.2 mg or more, 0.4 mg or more, 0.6 mg or more, or 0.7 mg or more.

The daily dose of telmisartan for a patient with hypertension is 20 mg to 80 mg (see the package insert of Micardis (registered trademark) tablets (Nippon Boehringer Ingelheim Co., Ltd.)). Accordingly, the daily dose of telmisartan to be used as an active ingredient of the pharmaceutical composition of the invention for inhibiting fibrosis in organs may be 16 mg or less, 8 mg or less, 4 mg or less, 2 mg or less, 1.6 mg or less, 1 mg or less, 0.8 mg or less, 0.6 mg or less, or 0.4 mg or less. The lower limit of the daily dose is not limited to a particular amount as long as the inhibitory effect on fibrosis is exerted, and may be, for example, 0.05 mg or more, 0.1 mg or more, 0.2 mg or more, or 0.3 mg or more.

The daily dose of olmesartan medoxomil for a patient with hypertension is 5 mg to 40 mg (see the package insert of OLMETEC (registered trademark) tablets (Daiichi Sankyo Company, Limited)). Accordingly, the daily dose of olmesartan medoxomil to be used as an active ingredient of the pharmaceutical composition of the invention for inhibiting fibrosis in organs may be 4 mg or less, 3 mg or less, 2 mg or less, 1 mg or less, 0.8 mg or less, 0.5 mg or less, 0.25 mg or less, 0.2 mg or less, or 0.1 mg or less. The lower limit of the daily dose is not limited to a particular amount as long as the inhibitory effect on fibrosis is exerted, and may be, for example, 0.04 mg or more, 0.06 mg or more, 0.08 mg or more, or 0.09 mg or more.

The daily dose of irbesartan for a patient with hypertension is 50 mg to 200 mg (see the package insert of AVAPRO (registered trademark) tablets (Dainippon Sumitomo Pharma Co., Ltd.)). Accordingly, the daily dose of irbesartan to be used as an active ingredient of the pharmaceutical composition of the invention for inhibiting fibrosis in organs may be 40 mg or less, 20 mg or less, 10 mg or less, 5 mg or less, 4 mg or less, 3 mg or less, 2.5 mg or less, 2 mg or less, 1.5 mg or less, or 1 mg or less. The lower limit of the daily dose is not limited to a particular amount as long as the inhibitory effect on fibrosis is exerted, and may be, for example, 0.4 mg or more, 0.6 mg or more, 0.8 mg or more, or 0.9 mg or more.

The daily dose of azilsartan for a patient with hypertension is 20 mg to 40 mg (see the package insert of AZILVA (registered trademark) tablets (Takeda Pharmaceutical Company Limited.)). Accordingly, the daily dose of azilsartan to be used as an active ingredient of the pharmaceutical composition of the invention for inhibiting fibrosis in organs may be 8 mg or less, 4 mg or less, 2 mg or less, 1 mg or less, 0.8 mg or less, 0.6 mg or less, 0.5 mg or less, or 0.4 mg or less. The lower limit of the daily dose is not limited to a particular amount as long as the inhibitory effect on fibrosis is exerted, and may be, for example, 0.05 mg or more, 0.1 mg or more, 0.2 mg or more, 0.3 mg or more, or 0.9 mg or more.

The pharmaceutical composition of the invention exerts inhibitory effect on fibrosis in organs. The term "fibrosis" as used herein includes the conditions of fibrosis, transition to the conditions of fibrosis, and the development, progression or aggravation of fibrosis. Biomechanics resides in all tissue organs and causes dynamic changes to these organs. Therefore, the subject organ of the invention is not particularly limited, and organs that may develop fibrosis can be the subject to be treated with the pharmaceutical composition of the invention. Examples of the subject organ may include, for example, liver, kidney, heart, lung, stomach, intestines, skeletal muscles, skin, etc. The subject organ is preferably liver, kidney, and heart, more preferably liver and kidney, particularly preferably liver.

Organ fibrosis for which the pharmaceutical composition of the invention is indicated may be any fibrosis induced by a change in biomechanics, such as hydrostatic pressure, osmotic pressure, tensile stress, compressive stress, etc. in an organ. Fibrosis induced by a biomechanical change in an organ is a condition in which when an abnormal change (increase or decrease) occurs in hydrostatic pressure, osmotic pressure, tensile stress, compressive stress, etc. in an organ, various types of cells perceive the mechanical change and then activate various biological signaling pathways to prompt tissue cells to produce an excessive amount of extracellular matrix, which then accumulates as fibrosis tissue. Fibrosis induced by a biomechanical change for which the pharmaceutical composition of the invention is indicated may be fibrosis induced by a change in hydrostatic pressure in an organ. Diseases associated with fibrosis induced by a change in hydrostatic pressure in an organ may include liver failure, heart failure, kidney failure, lung fibrosis, myofibrosis, and skin cicatrization. Diseases associated with fibrosis induced by a change in tensile stress and/or compressive stress in an organ may include skin cicatrization, fibrosis of cardiac muscle tissue, etc.

The pharmaceutical composition of the invention can be formulated by appropriately blending an angiotensin II receptor antagonist as an active ingredient with a pharmaceutically acceptable carrier or additive in accordance with a known production method for pharmaceutical formulations (e.g., the methods described in the Japanese pharmacopoeia, etc.). Specific examples of such a pharmaceutical formulation include oral or parenteral formulations, such as tablets (including sugar-coated tablets, film-coated tablets, sublingual tablets, orally disintegrating tablets, buccal tablets, etc.), pills, powders, granules, capsules (including soft capsules and microcapsules), troches, syrups, liquids, emulsions, suspensions, controlled release formulations (e.g., fast release formulations, sustained release formulations, sustained release microcapsules, etc.), aerosols, films (e.g., orally disintegrating films, oral mucosal adhesive films, etc.), injections (e.g., subcutaneous injection, intravenous injection, intramuscular injection, intraperitoneal injection, etc.), intravenous infusions, transdermal formulations, ointments, lotions, patches, suppositories (e.g., rectal suppository, vaginal suppository, etc.), pellets, transnasal formulations, transpulmonary formulations (inhalants), eye drops, etc. The blending ratio of a carrier or additive can be determined as appropriate based on the amount usually used in the pharmaceutical field. The carrier or additive that can be blended is not limited to a particular one, and examples thereof include various types of carriers, such as water, physiological saline, other aqueous solvents, or aqueous or oily bases; and various types of additives, such as excipients, binders, pH adjusting agents, disintegrants, absorption enhancers, lubricants, colorants, flavor improvers and fragrances.

Additives that can be blended into tablets, capsules, etc., may include, for example, binders such as gelatin, corn starch, tragacanth, or gum arabic; excipients such as crystalline cellulose; swelling agents such as corn starch, gelatin, or alginic acid; lubricants such as magnesium stearate; sweeteners such as sucrose, lactose, or saccharin; flavoring agents such as peppermint, wintergreen oil, or cherry; etc. When the formulation unit is a capsule, a liquid carrier such as fats and oils can be further blended into the formulation, in addition to the ingredients of the above types. A sterile composition for injection can be prepared according conventional formulation procedures (for example, by dissolving or suspending the active ingredient in a solvent, such as water for injection or a natural vegetable oil). An aqueous solution for injection may be, for example, physiological saline or an isotonic solution containing glucose or other auxiliary agents (e.g., D-sorbitol, D-mannitol, sodium chloride, etc.). The aqueous solution for injection may also contain an appropriate solubilizing agent, including, for example, alcohols (ethanol etc.), polyalcohols (propylene glycol, polyethylene glycol, etc.), and nonionic surfactants (polysorbate 80, HCO-50, etc.). The oily solution for injection may be, for example, sesame oil, soybean oil, or the like, and may also contain a solubilizing agent such as benzyl benzoate and benzyl alcohol. Additionally, the composition for injection may further contain a buffering agent (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), etc.

The angiotensin II receptor antagonist used as an active ingredient of the pharmaceutical composition of the invention is a substance that has already been used in the clinical setting over many years. Therefore, the active ingredient can be safely administered to humans or other mammals (e.g., rats, mice, rabbits, sheep, pigs, cows, cats, dogs, monkeys, etc.). The amount of the active ingredient in a formulation may vary with the dosage form, the mode of administration, the carrier used, etc., but is usually 0.01 to 100% (w/w), or may be 0.1 to 95% (w/w), relative to the total amount of the formulation.

The pharmaceutical composition of the invention is used so that the angiotensin II receptor antagonist as an active ingredient is administered at a lower dose than the dose of the angiotensin II receptor antagonist used for the original indication as an antihypertensive. Therefore, the pharmaceutical composition of the invention has an advantageous effect of inhibiting fibrosis in an organ at a dose that exert no or only mild antihypertensive effect. Accordingly, the pharmaceutical composition of the invention is very advantageously and safely used even for a subject with a blood pressure in a normal range and a subject with hypotension. Since the active ingredient is administered at a lower dose, peripheral vessels or blood flow will not be substantially affected. Therefore, tissue/organ injury due to reduction in local tissue blood flow due to the active ingredient at a usual dose or a poisonous side effect by other medicines will be minimized. In this manner, the pharmaceutical composition of the invention is advantageous for long-term administration.

### EXAMPLES

The present invention will be described in more detail below with reference to Examples, but the present invention is not limited thereto.

### Example 1: Evaluation using partial inferior vena cava ligation model mice

### 1. Materials and methods

### (1) Animals

C57BL/6 mice (10-week-old, CLEA) were used. All animal experiments were approved by the Institutional Animal Care and Use Committee of Nagasaki University, and carried out in accordance with the institutional guidelines.

### (2) Partial inferior vena cava ligation model and losartan treatment

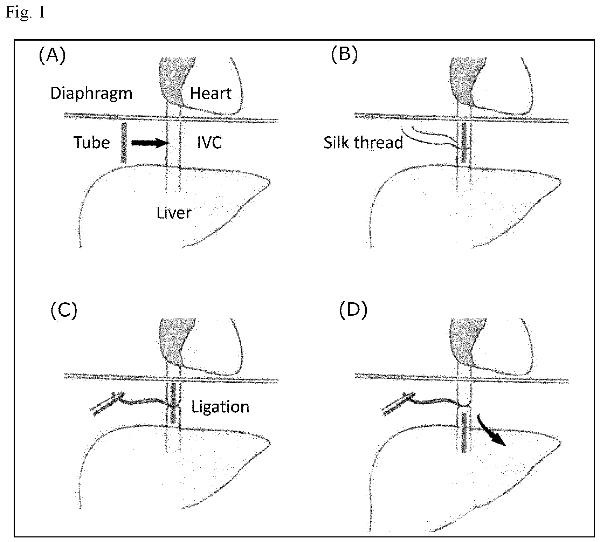

Partial inferior vena cava ligation was performed as described in Simonetto et al. (Hepatology 61: 648-659, 2015. doi: 10.1002/hep.27387). The procedure of partial inferior vena cava ligation is shown in Fig. 1. Briefly, the abdomen of mouse was opened under anesthesia, and the suprahepatic inferior vena cava (IVC) was exposed after dividing the falciform ligament ((A) in Fig. 1). IVC was separated and a small tube (0.9 mm diameter) was placed along with the anterior surface of IVC ((B) in Fig. 1). The tube was tightly ligated together with the IVC ((C) in Fig. 1), and immediately after that, the tube was gently removed ((D) in Fig. 1). The abdomen was then closed with a suture of the peritoneal muscle/fascia followed by closure of the skin. Removing the 0.9 mm diameter tube from IVC ligation was expected to reduce the cross-sectional area of IVC by about 80% based on Simonetto et al.

After all procedures, mice were divided into Losartan group (n = 6), IVCL group (n = 6) and Sham group (n = 6). Losartan group was given losartan (FUJIFILM Wako Pure Chemical Corporation) in their drinking water at a final concentration of 2.3 mg/L, which providing an estimated daily dose of 0.5 mg/kg. IVCL group was given drinking water without addition of any drug. Treatment was continued until the cessation of the experiments. Sham group received a sham operation including all above steps with the exception of partial IVC ligation, and was given drinking water without addition of any drug.

The inventors investigated many previous studies reporting experimental results of losartan administration to experimental animals and found that most of the studies use around 3 to 30 mg/kg daily dose in rats and mice. The inventors chose a low-dose losartan of 0.5 mg/kg/day in this Example, which provides a sufficiently lower daily dose compared to that in the conventional art. Dose conversion from an experimental animal dose to a human dose (Human equivalent dose (HED)) is commonly calculated on the basis of the ratio of body surface area between experimental animals and humans to estimate a dose in humans anticipated to provide the same degree of effect that observed in animals at a given dose. RED can be determined by reference to Guidance for Industry Estimating the Maximum Safe Starting Dose in Initial Clinical Trials for Therapeutics in Adult Healthy Volunteers published by the United States Food and Drug Administration (FDA), or the like. Extrapolation of HED from a mouse dose is usually performed using a factor of 12.3 when no comparison data are available. The dose of losartan in mice in this Example at 0.5 mg/kg/day is estimated to be 0.04 mg/kg/day in humans. This dose is much lower than the clinically recommended dose of losartan for patients with hypertension (the minimum recommended dose of losartan for a human weighing 60 kg is 20 mg/day).

### (3) Tissue sampling and serum analyses

Mice were euthanized 8 weeks postoperatively. Prior to sacrifice, the body weight was measured, and whole blood was collected from IVC and serum was separated. The levels of serum ALT and AST were measured. The suprahepatic IVC was then transected and the liver was perfused with PBS through the portal vein to remove circulating blood and fibrinogen. The spleen and liver were harvested and weighed, and the spleen/body and liver/body weight ratios were calculated. Liver tissues were fixed in 4% paraformaldehyde and embedded into paraffin block. Tissue sections of about 5 µm thickness were used for histological analysis.

### (4) Masson's trichrome staining

Liver tissue sections were deparaffinized and rehydrated. Masson's trichrome staining was performed using Trichrome Stain (Masson) Kit (Sigma-Aldrich) according to the manufacturer's instructions, and then observed under an optical microscope (IX71, Olympus).

### (5) Immunohistochemistry

Immunohistochemistry analysis on the liver tissues was performed to understand the relevant mechanism. Briefly, liver tissue sections were blocked with 10% bovine serum albumin, and then incubated with primary antibodies (Table 1) overnight at 4°C, followed by incubation with secondary antibodies (Table 2) for 1 hour at room temperature in the dark. Sections were then mounted with medium containing DAPI. Apoptotic cells were detected by the usage of TUNEL staining kit (Abcam, #ab66108) according to the manufacturer's instructions. Briefly, sections were incubated with DNA labeling solution at 37°C for 60 min, followed by the addition of PI/RNase A solution for 30 min. Immunofluorescences of stained liver tissues were detected using a fluorescence microscope (FV10i, Olympus). For each staining, at least 10 images were taken from randomly selected fields at 60x magnification, and the mean fluorescence intensity was measured by ImageJ software.

**Table 1. Primary antibodies**

| | | | |
|---|---|---|---|
| Antibody | Manufacturer | Catalog number | |
| Mouse Anti-RhoA | SantaCruz | SC-418 | AB_628218 |
| Rabbit Anti-ROCK1 | Abcam | ab97592 | AB_10688425 |
| Rabbit Anti-ROCK2 | Abcam | ab71598 | AB_1566688 |
| Rabbit Anti-Collagen \| | Abcam | ab34710 | AB_731684 |
| Rabbit Anti-TGF-β1 | Abcam | ab92486 | AB_10562492 |
| Rabbit Anti-α-Smooth Muscle Actin | CST | 19245 | AB_2734735 |
| Rabbit Anti-Phospho-myosin light chain 2 | CST | 3674 | AB_2147464 |
| Rabbit Anti AGTR1 Polyclonal | Invitrogen | PAS-20812 | AB_11157209 |

**Table 2. Secondary antibodies**

| | | | |
|---|---|---|---|
| Antibody | Manufacturer | Catalog number | RRID |
| Goat Anti-rabbit (Alexa Flour 488) | Invitrogen | A32731 | AB_2633280 |
| Goat Anti-mouse (Alexa Flour 488) | Invitrogen | A11029 | AB_138404 |

### (6) Statistical analysis

All the results were presented as mean ± SD. Statistical significance was determined by one-way analysis of variance (ANOVA) followed by Turkey's test. P value < 0.05 was accepted as significant. GraphPad Prism 8 software was used for statistical analysis.

### 2. Result

### (1) Body weight, organ weights and serum analyses

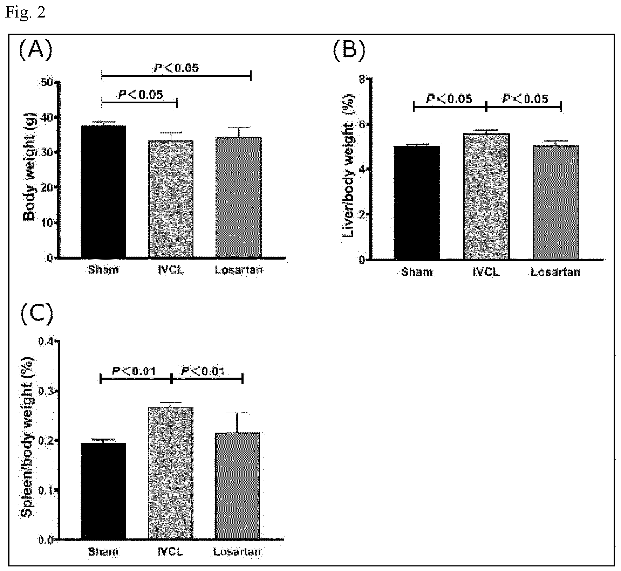

The body weight and organ weights are shown in Fig. 2. Panel (A) shows the body weight of mice 8 weeks postoperatively. Panel (B) shows the liver/body weight ratio, and panel (C) shows the spleen/body weight ratio. The liver/body and spleen/body weight ratios were significantly higher in IVCL group than Sham group, suggesting liver congestion and damage in mice received partial IVC ligation. However, the liver/body and spleen/body weight ratios of Losartan group were kept at comparable levels as those of mice in Sham group.

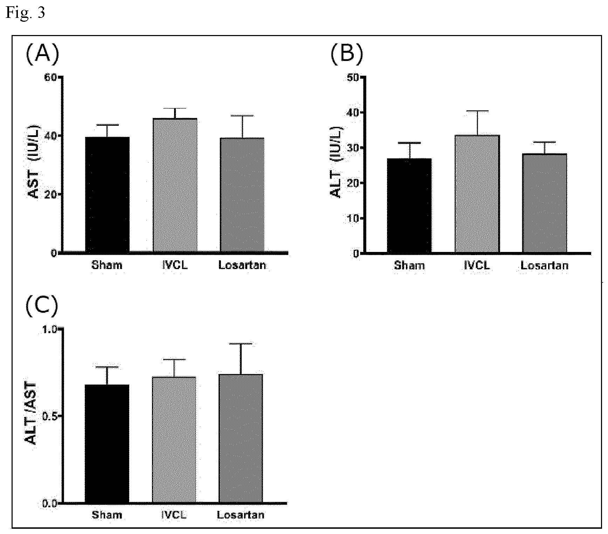

The results of serum analyses are shown in Fig. 3. Panel (A) shows the levels of AST, panel (B) shows the levels of ALT, and panel (C) shows ALT/AST ratios. The levels of ALT and AST in serum were not significantly different among all groups.

### (2) Fibrosis (Masson's trichrome staining)

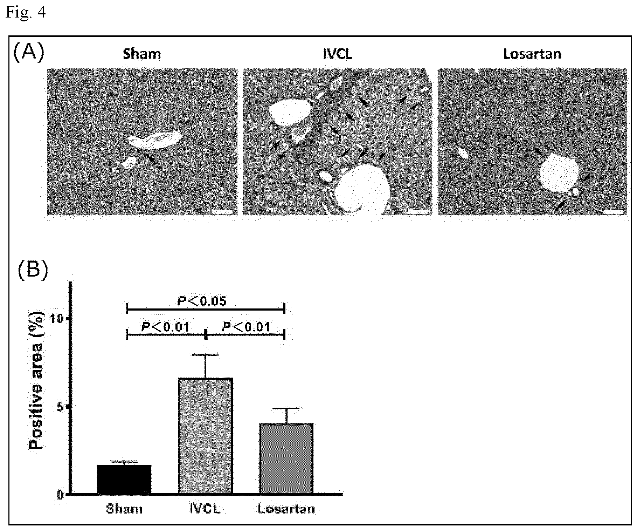

Masson's trichrome staining of the liver tissues are shown in Fig. 4. Panel (A) shows representative images of the staining in each group, and arrows indicate fibrotic regions. Panel (B) shows comparison of the percentages of positive area of Masson's trichrome staining of the groups by image analysis. Liver tissues from Sham group had normal lobular architecture with few of collagen deposition. The fibrotic area was obviously increased in IVCL group, and the lobular architecture surrounding the fibrotic area was obviously disturbed. However, fibrotic area was significantly decreased in Losartan group compared to that in IVCL group, and changes in the lobular architecture were effectively alleviated.

### (3) Apoptosis

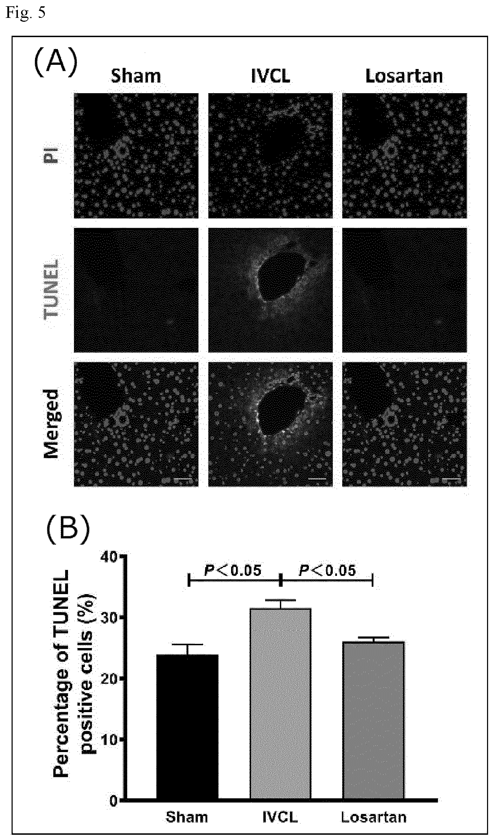

Apoptotic cells observed in the liver tissues are shown in Fig. 5. Panel (A) shows representative images of TUNEL staining in each group. Panel (B) shows comparison of the percentages of positive area of TUNEL staining by image analysis. Compared to Sham group, the percentage of apoptotic cells was significantly increased in IVCL group, but was not significantly changed in Losartan group.

### (4) Expression of α-SMA

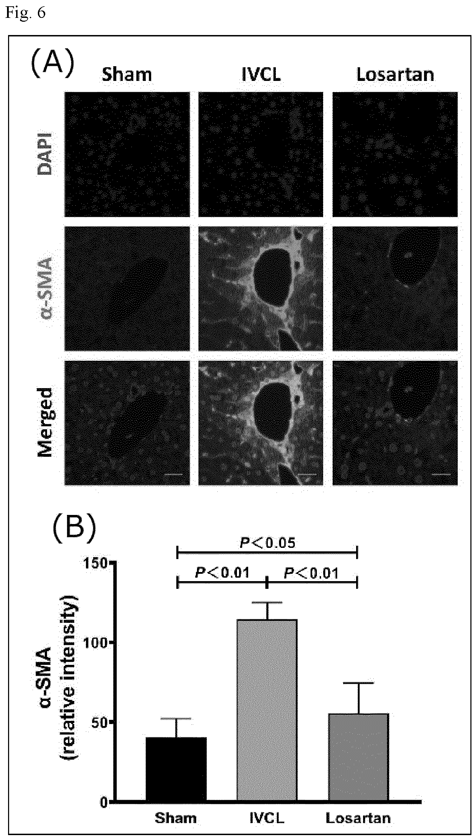

Immunostaining of α-SMA in the liver tissues is shown in Fig. 6. α-SMA is a marker for activated hepatic stellate cells. Panel (A) shows representative images of immunostaining of α-SMA in each group. Panel (B) shows comparison of signal intensity of α-SMA expression by image analysis. The expression of α-SMA was more extensively detected in the liver tissues of IVCL group than that of Sham group, and a significant difference in the signal intensity was observed between the groups. In contrast to the enhanced expression of α-SMA in IVCL group, the expression of α-SMA was significantly reduced in Losartan group.

### (5) Expression of RhoA, ROCK1, ROCK2 and p-MLC2

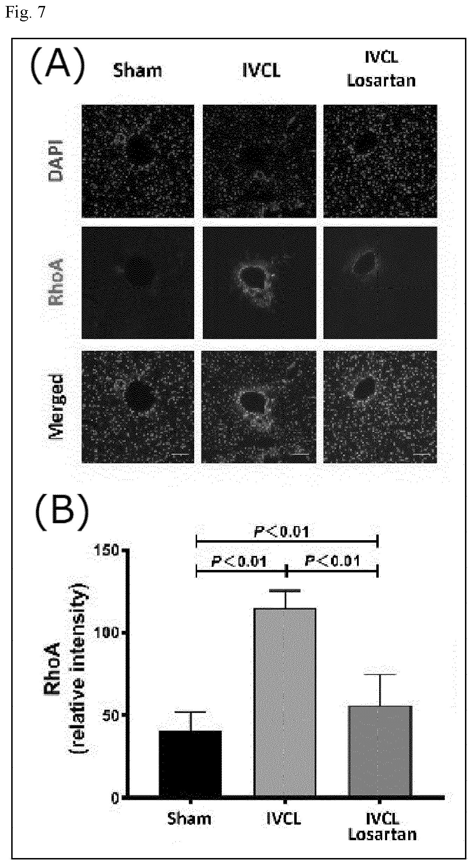

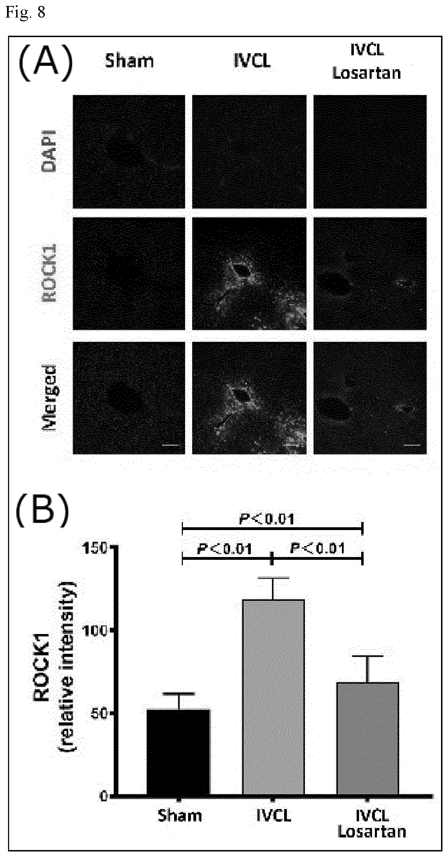

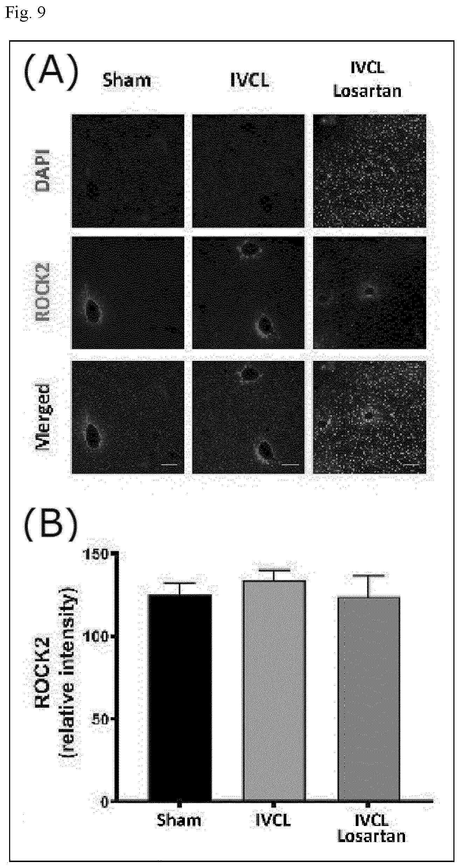

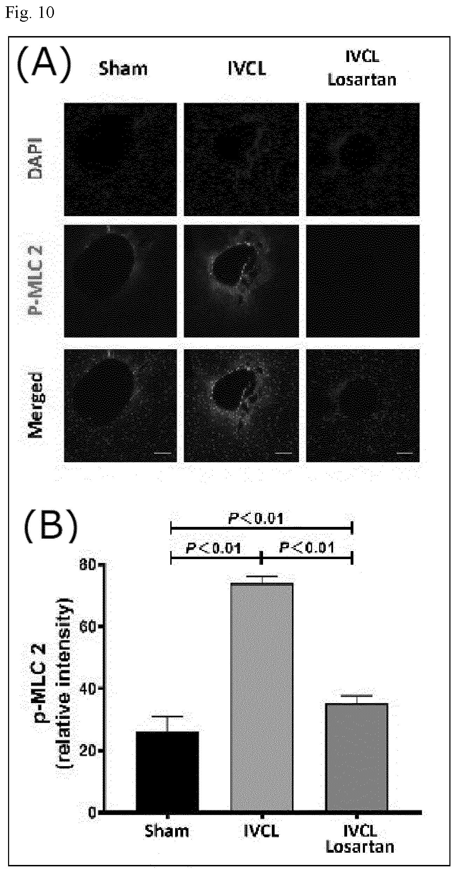

The inventors also investigated the expression of several molecules related to the RhoA/ROCK signaling pathway because of its central role on mechanotransduction. Immunostaining of RhoA, ROCK1, ROCK2 and p-MLC2 in the liver tissues is shown in Figs. 7, 8, 9 and 10, respectively. In Figs. 7 to 10, panels (A) show representative images of immunostaining in each group, and panels (B) show comparison of signal intensity of expression by image analysis. Expression of ROCK2 in the liver tissues was not substantially changed in all groups (Fig. 9). Compared to Sham group, RhoA and ROCK1 were significantly upregulated in liver cells surrounding the portal vein in IVCL group, but were significantly reduced in Losartan group (Figs. 7 and 8). Expression of p-MLC2, a downstream molecule of ROCK, was also significantly upregulated in IVCL group compared to that in Sham group, but was effectively attenuated in Losartan group (Fig. 10).

### (6) Summary

All these findings from in vivo experiments suggest that low-dose losartan is able to alleviate liver fibrosis by interfering the RhoA/ROCK signaling pathway.

### Example 2: Evaluation using human hepatic stellate cells

### 1. Materials and methods

### (1) Human hepatic stellate cells (HSCs)

Primary human HSCs were purchased from ScienCell Research Laboratories. Cells were expanded by using stellate cell medium (ScienCell Research Laboratories) in a humidified incubator under 5% CO₂ and 95% air at 37°C. The third passaged cells were used for the following experiments.

### (2) Ex vivo hydrostatic pressure loading test in human HSCs

A pneumatic pressurizing system (Strex. Inc) was used to induce hydrostatic pressure. Briefly, HSCs were seeded in 60-mm diameter culture dishes (5 × 10⁴ cells/dish) and 4-well culture chamber slides (2 × 10⁴ cells/well). Cells were incubated for 3 days to form about 70% confluent, and then half of the culture dishes and slides were randomly selected to be loaded by 50 mmHg hydrostatic pressure for 24 hours, with or without the addition of 10 nM losartan in medium. The inventors used 50 mmHg for experiments based on their preliminary experiments and a previous report (Yoshino et al., Commun Biol 3: 152, 2020, Chen et al., Int J Biol Sci 15: 2509-2521, 2019). As controls, non-hydrostatic pressure-loaded groups were provided for each of Losartan addition group and non-Losartan addition group.

### (3) RT-qPCR

RT-qPCR was performed to evaluate the expression of RHOA, ROCK1, ROCK2, ACTA2, TGFB1, and COL1A1. Briefly, total RNA was isolated from HSCs using Quick-RNA MicoroPrep Kit, and 1.25 µg of RNA was reverse-transcribed using SuperScript VII,O cDNA Synthesis Kit (Thermo Fisher Scientific). Quantitative PCR was carried out with SYBR Green real-time PCR Master Mix (Toyobo). The reactions were performed on a CFX96 real-time PCR System (BIO-RAD). The primer sequences are shown below. GAPDH was used for normalization.

RHOA Forward: CAGAAAAGGGACCCCAGAA (SEQ ID NO: 1)
RHOA Reverse: GCAGCTGCTCTCGTAGCCATTTC (SEQ ID NO: 2)
ROCK1 Forward: AACATGCTGCTGGATAAATCTGG (SEQ ID NO: 3)
ROCK1 Reverse: AGGAAGGCATGGTACGATGTGATACA (SEQ ID NO: 4)
ROCK2 Forward: TCAGAGGTCTACAGATGAAGGC (SEQ ID NO: 5)
ROCK2 Reverse: CCAGGGGCTATTGGCAAAGG (SEQ ID NO: 6)
ACTA2 Forward: GTGACGAAGCACAGAGCAAA (SEQ ID NO: 7)
ACTA2 Reverse: CTTTTCCATGTCGTCCCAGT (SEQ ID NO: 8)
COL1A1 Forward: CCCACCAATCACCTGCGTACAGA (SEQ ID NO: 9)
COL1A1 Reverse: TTCTTGGTCGGTGGGTGACTCTGA (SEQ ID NO: 10)
SMAD2 Forward: TCTTGATGGTCGTCTCCAGGTA (SEQ ID NO: 11)
SMAD2 Reverse: GAGGCGGAAGTTCTGTTAGGAT (SEQ ID NO: 12)
GAPDH Forward: GACTCATGACCACAGTCCATGC (SEQ ID NO: 13)
GAPDH Reverse: AGAGGCAGGGATGATGTTCTG (SEQ ID NO: 14)
AT1R Forward: TCAACAAAAATGAGCACGCTTT (SEQ ID NO: 15)
AT1R Reverse: AAACATGGTGCAGGCTTCTTG (SEQ ID NO: 16)

### (4) Immunohistochemistry

HSCs were fixed in 4% paraformaldehyde before blocked by 10% bovine serum albumin. The primary antibodies and secondary antibodies used were listed in Tables 1 and 2. After overnight incubation with the primary antibodies, cells were incubated with secondary antibodies for 1 hour at room temperature, and then mounted with medium containing DAPI. F-actin fibers were stained with TRITC-conjugated phalloidin in mounting medium. Immunofluorescences of stained cells were detected using a fluorescence microscope (FV10i, Olympus). For each staining, 10 images were taken from randomly selected fields at 60x magnification, and the mean fluorescence intensity was measured by ImageJ software.

### (5) Statistical analysis

Statistical analysis was performed in the same manner as in Example 1.

### 2. Result

### (1) RT-qPCR of AT1R, ACTA2, TGFB1 and COL1A1

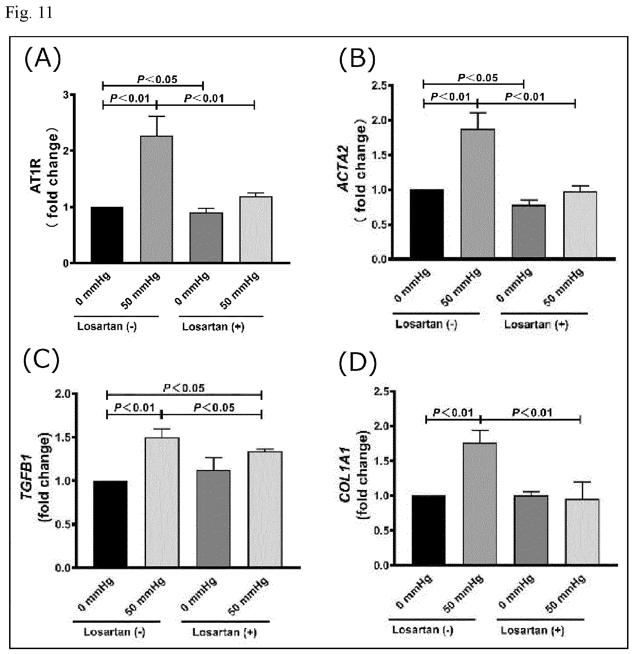

The results of RT-qPCR of AT1R, ACTA2, TGFB1 and COL1A1 are shown in Fig. 11. Panel (A) shows the results of AT1R, panel (B) shows the results of ACTA2 (the gene encoding α-SMA), panel (C) shows the results of TGFB1 (the gene encoding TGF-β1), and panel (D) shows the results of COL1A1 (the gene encoding type 1 collagen). Hydrostatic pressure-loaded group without addition of losartan showed significant upregulation of the expression of AT1R, ACTA2, TGFB1 and COL1A1 genes compared to the non-hydrostatic pressure-loaded group. However, the expression of AT1R, ACTA2, TGFB1 and COL1A1 genes in the hydrostatic pressure-loaded group with addition of losartan was not significantly different from that of the non-hydrostatic pressure-loaded group, but significantly decreased compared to the hydrostatic pressure-loaded group without addition of losartan.

### (2) Immunohistochemistry of AT1R

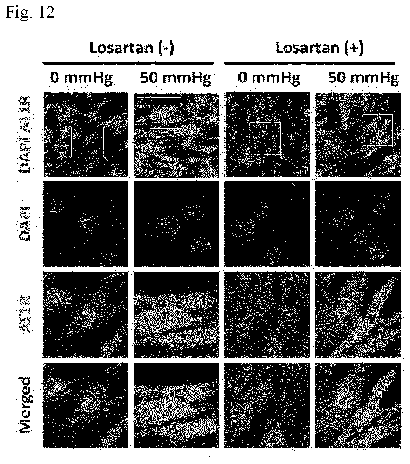

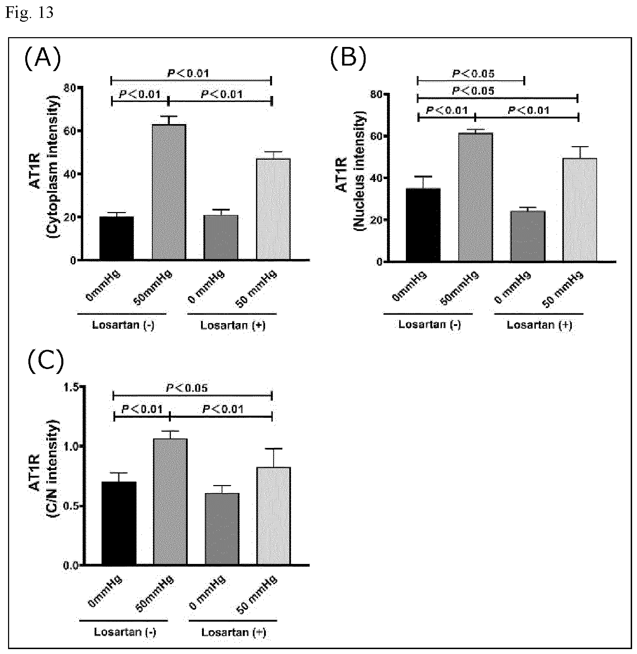

Immunohistochemistry of AT1R is shown in Figs. 12 and 13. Fig. 12 shows representative images of immunostaining in each group. In Fig. 13, panel (A) shows comparison of signal intensity of AT1R expression in the cytoplasm by image analysis, panel (B) shows comparison of signal intensity of AT1R expression in the nucleus by image analysis, and panel (C) shows comparison of cytoplasm signal intensity/nucleus signal intensity. Hydrostatic pressure-loaded group without addition of losartan showed significant upregulation of the expression of AT1R at the protein level in the cytoplasm and nucleus. However, the expression of the gene in the cytoplasm and nucleus in the hydrostatic pressure-loaded group with addition of losartan was not significantly different from that of the non-hydrostatic pressure-loaded group, but significantly decreased compared to the hydrostatic pressure-loaded group without addition of losartan.

### (3) Immunohistochemistry of α-SMA, TGF-β1 and type 1 collagen

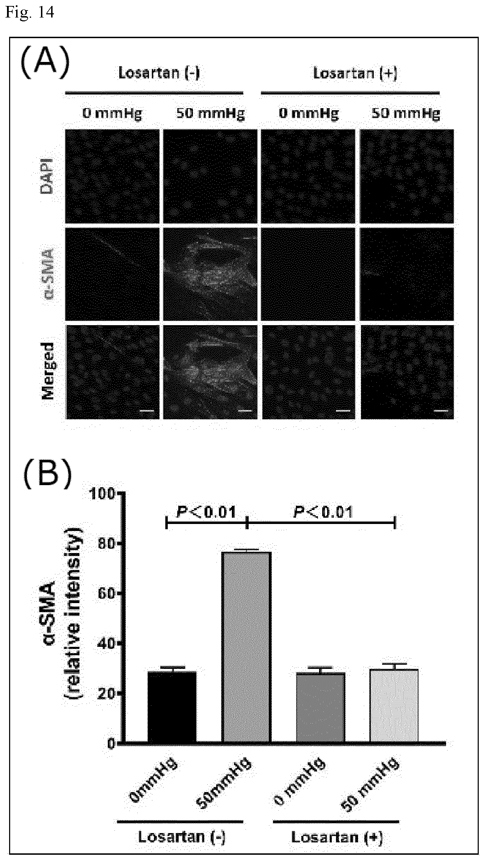

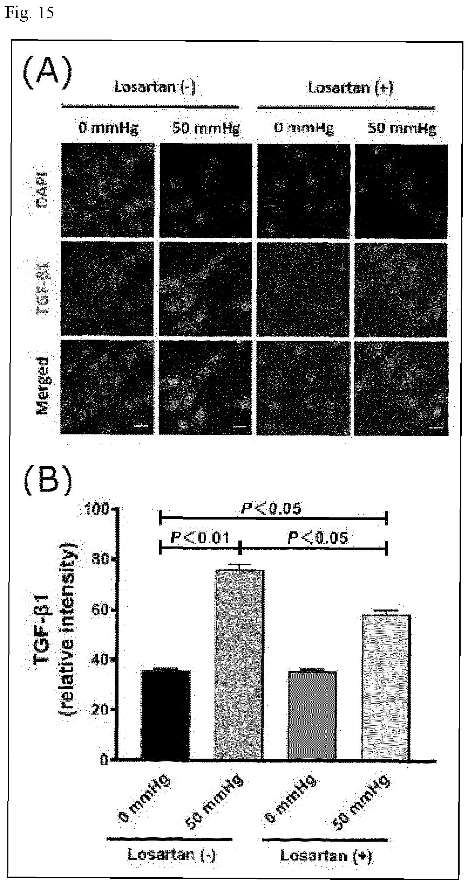

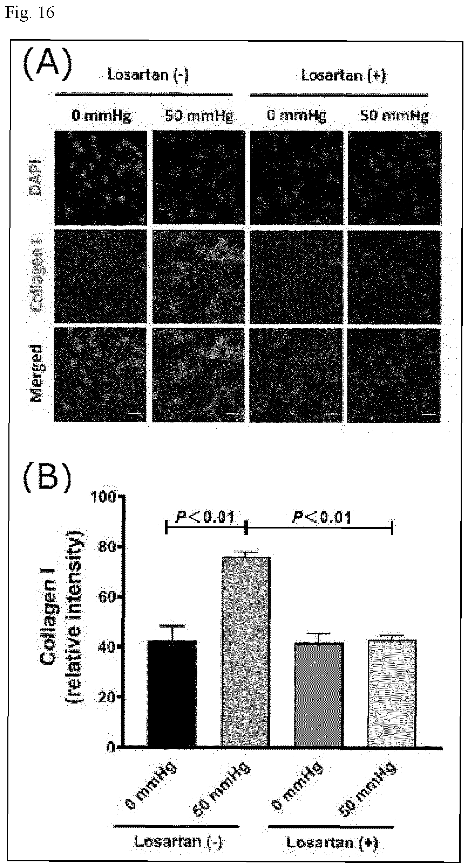

Immunohistochemistry results of α-SMA, TGF-β1 and type 1 collagen are shown in Figs. 14, 15 and 16, respectively. In Figs. 14 to 16, panels (A) show representative images of immunostaining in each group, and panels (B) show comparison of signal intensity of expression by image analysis. As with the results in RT-qPCR, the hydrostatic pressure-loaded group without addition of losartan showed significant upregulation of the expression of α-SMA, TGF-β1 and type 1 collagen at the protein level. However, the expression of α-SMA, TGF-β1 and type 1 collagen in the hydrostatic pressure-loaded group with addition of losartan was not significantly different from that of the non-hydrostatic pressure-loaded group, but significantly decreased compared to that of the hydrostatic pressure-loaded group without addition of losartan.

### (4) RT-qPCR of RhoA, ROCK1 and ROCK2

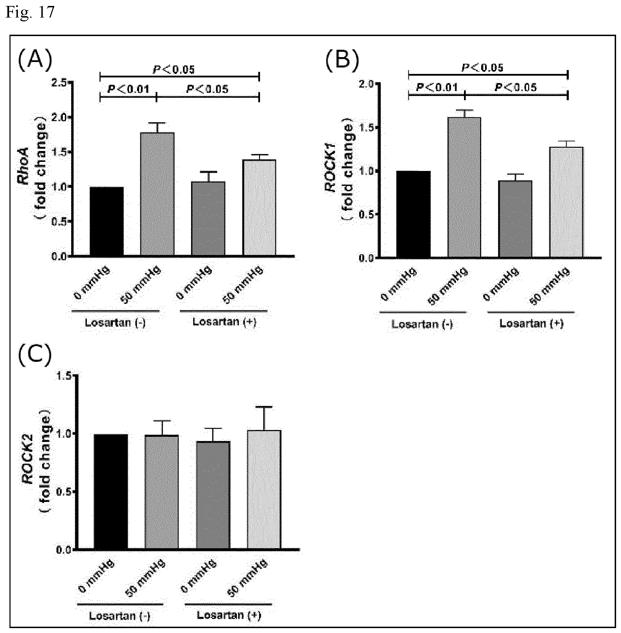

The results of RT-qPCR of RhoA, ROCK1 and ROCK2 are shown in Fig. 17. Panels (A), (B) and (C) show the results of RT-qPCR of RhoA, ROCK1 and ROCK2, respectively. Hydrostatic pressure-loaded group without addition of losartan showed significant upregulation of the expression of RhoA and ROCK1 genes compared to that of the non-hydrostatic pressure-loaded group. However, the expression of RhoA and ROCK1 genes in the hydrostatic pressure-loaded group with addition of losartan was not significantly different from that of the non-hydrostatic pressure-loaded group, but significantly decreased compared to that of the hydrostatic pressure-loaded group without addition of losartan. However, no significant change in the expression of ROCK2 gene was observed in the hydrostatic pressure-loaded group with addition of losartan.

### (5) Immunohistochemistry of RhoA, ROCK1 and ROCK2

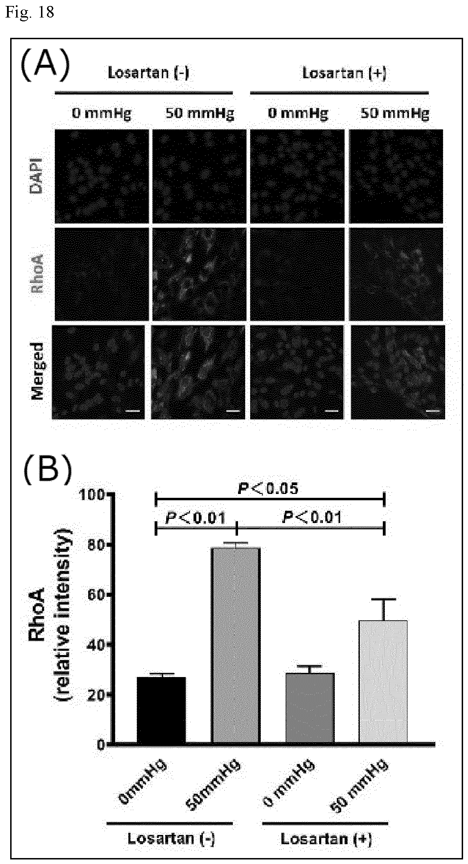

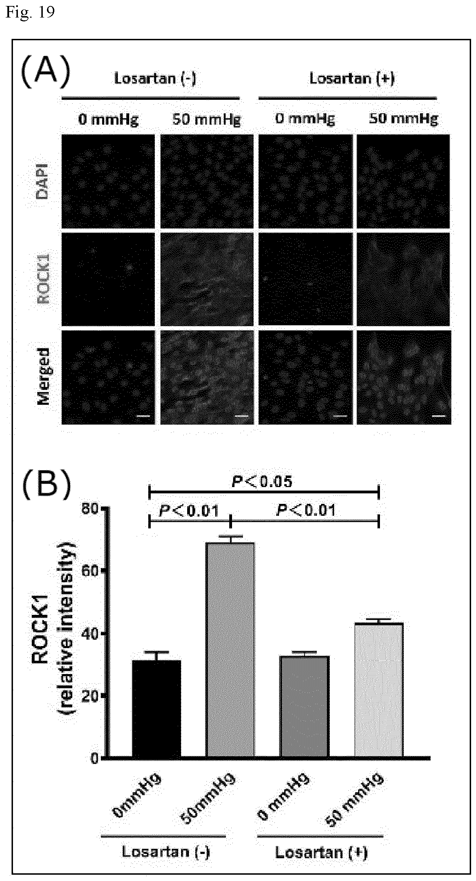

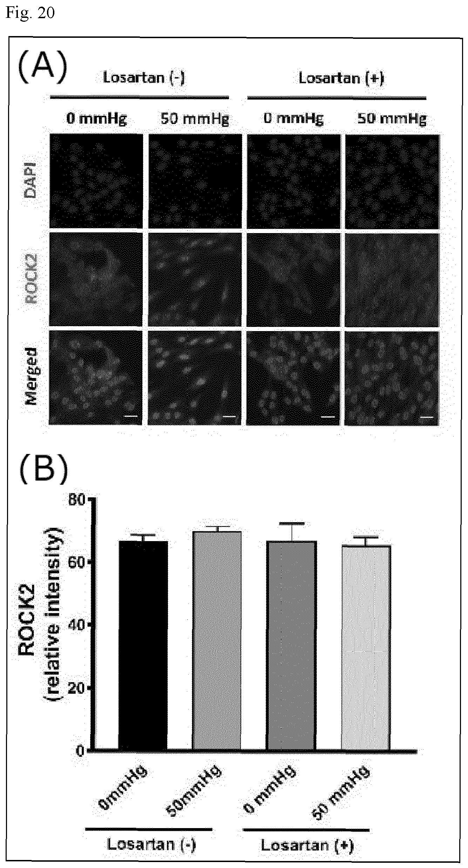

Immunohistochemistry results of RhoA, ROCK1 and ROCK2 are shown in Figs. 18, 19 and 20, respectively. In Figs. 18 to 20, panels (A) show representative images of immunostaining in each group, and panels (B) show comparison of signal intensity of expression by image analysis. As with the results in RT-qPCR, the hydrostatic pressure-loaded group without addition of losartan showed significant upregulation of the expression of RhoA and ROCK1 at the protein level. However, the expression of RhoA and ROCK1 in the hydrostatic pressure-loaded group with addition of losartan was not significantly different from that of the non-hydrostatic pressure-loaded group, but significantly decreased compared to that of the hydrostatic pressure-loaded group without addition of losartan. However, no significant change in the expression of ROCK2 gene was observed in the hydrostatic pressure-loaded group with addition of losartan.

### (6) Phalloidin staining for F-actin fibers and immunohistochemistry of p-MLC2

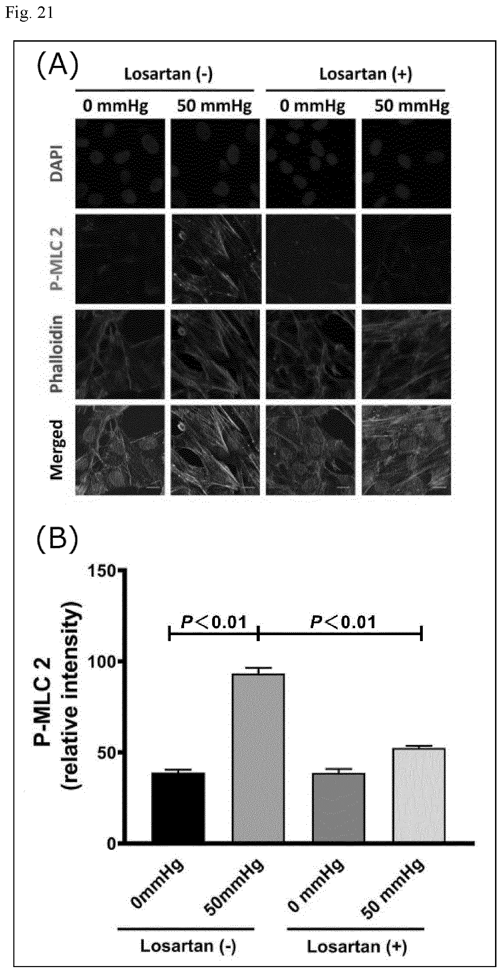

The results of the staining are shown in Fig. 21. Panel (A) shows representative images of the staining in each group, and panel (B) shows comparison of signal intensity of p-MI,C2 by image analysis. Phalloidin staining for F-actin revealed that the hydrostatic pressure-loaded group without addition of losartan exhibited thick actin bundles and formed a dense parallel network. However, the formation of actin stress fibers was effectively reduced in the hydrostatic pressure-loaded group with addition of losartan. Additionally, the hydrostatic pressure-loaded group without addition of losartan showed significant upregulation of the expression of p-MI,C2. However, the expression of p-MLC2 in the hydrostatic pressure-loaded group with addition of losartan was not significantly different from that of the non-hydrostatic pressure-loaded group, but significantly decreased compared to that of the hydrostatic pressure-loaded group without addition of losartan.

### (7) Summary

Together, these ex vivo experimental data indicate that losartan is able to effectively alleviate the hydrostatic pressure-induced alternation of mechanotransduction properties of HSCs.

### Example 3: Evaluation using unilateral ureteral obstruction (UUO)-induced renal fibrosis model mice

### 1. Materials and methods

### (1) UUO-induced renal fibrosis model and losartan treatment

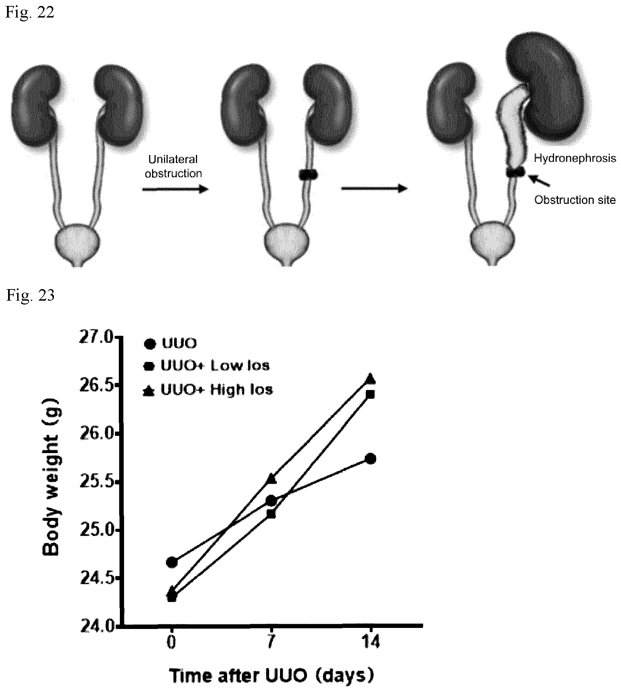

C57BL/6 mice (8-week-old, CLEA) were used. The procedure of unilateral ureteral obstruction (UUO) is shown in Fig. 22. The abdomen of mouse was opened under general anesthesia and the left ureter was ligated. The right ureter was not ligated and the right kidney served as a control. The surgical wound was then closed with a single-layer suture of the peritoneum, muscular layer and skin followed by closure of the surgical wound. UUO-induced renal fibrosis model is known to manifest the symptoms of hydronephrosis in the ligated kidney and then develop interstitial fibrosis caused by interstitial deposition of type IV collagen as well as apoptosis of tubular cells at day 3 post-UUO surgery. Post-UUO surgery mice were divided into three groups: low-dose losartan group (1 mg/kg, n = 3), high-dose losartan group (3 mg/kg, n = 3) and no losartan administration group (n = 3). Low-dose losartan group was given losartan (FUJIFILM Wako Pure Chemical Corporation) in their drinking water at a final concentration of 4.6 mg/L, which providing an estimated daily dose of 1 mg/kg. High-dose losartan group was given losartan in their drinking water at a final concentration of 13.8 mg/L, which providing an estimated daily dose of 3 mg/kg. No losartan administration group was given drinking water without addition of losartan.

### (2) Body weight measurement, tissue sampling and serum analyses

Body weight was measured prior to UUO surgery and 1 and 2 weeks post-surgery. Mice were euthanized 2 weeks post-surgery. Prior to sacrifice, whole blood was collected from IVC and serum was separated. The levels of serum BUN and CRE were measured. The left and right kidneys were then harvested and photographed. Kidneys were fixed in 4% paraformaldehyde and embedded into paraffin block. Tissue sections of about 5 µm thickness were used for histological analysis.

### (3) Masson's trichrome staining and Hematoxylin and Eosin staining

Tissue sections were deparaffinized and rehydrated. Masson's trichrome staining was performed using Trichrome Stain (Masson) Kit (Sigma-Aldrich) according to the manufacturer's instructions. Hematoxylin and Eosin (HE) staining was performed by a conventional method. Tissue specimens were observed under with an optical microscope (IX71, Olympus).

### (4) Statistical analysis

Statistical analysis was performed in the same manner as in Example 1.

### 2. Result

### (1) Body weight and kidney

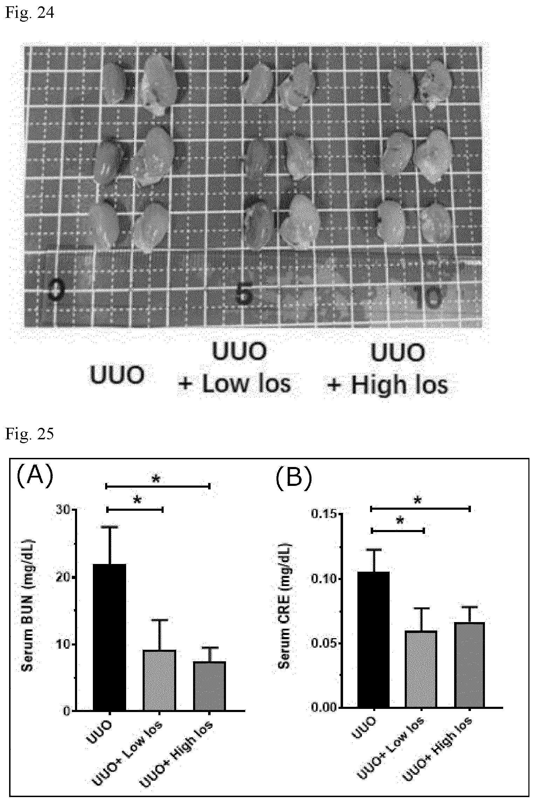

Changes in the body weight of mice in each group are shown in Fig. 23. The photograph of the left and right kidneys harvested from mice in each group is shown in Fig. 24. No significant change was observed in the body weight of mice among all groups at any measurement time points. The left kidneys of the control group were swollen due to hydronephrosis, but swelling of the left kidneys of low-dose losartan group and high-dose losartan group was significantly inhibited.

### (2) Serum analyses

The results of serum analyses are shown in Fig. 24. Panel (A) shows the levels of BUN and panel (B) shows the levels of CRE. The levels of BUN and CRE in low-dose losartan group and high-dose losartan group were significantly decreased compared to the control group.

### (3) Fibrosis (Masson's trichrome staining and HE staining)

Masson's trichrome staining and HE staining of the kidney tissues are shown in Fig. 25. Panel (A) shows representative images of staining in each group. The upper row shows the images of Masson's trichrome staining, and the lower row shows the images of HE staining. The images in the first column from the left (CON) show the right kidney of the control group, and the images in the second column from the left (UUO) show the left kidney of the control group. Panel (B) shows comparison of the percentages of positive area (indicated by arrows in the images in panel (A)) of Masson's trichrome staining in the groups by image analysis. The right kidney of the control group (CON) had almost no positive area of Masson's trichrome staining. The left kidney of the control group (UUO) indicates significant increase of fibrosis area, whereas low-dose losartan group and high-dose losartan group indicates significant decreasing of fibrosis area.

### (4) Summary

Together, these ex vivo experimental data suggest that low-dose losartan is able to alleviate kidney fibrosis.

The present invention is not limited to each of the embodiments and Examples as described above, and various modifications are possible within the scope of the claims. Embodiments obtainable by appropriately combining the technical means disclosed in the different embodiments of the present invention are also included in the technical scope of the present invention. The contents of the scientific literature and the patent literature cited herein are hereby incorporated by reference in their entirety.

## Claims

1. A pharmaceutical composition for inhibiting fibrosis in an organ, comprising an angiotensin II receptor antagonist as an active ingredient, wherein the composition is used so that the angiotensin II receptor antagonist is administered at a daily dose that is 0.2 times or less the daily dose of the angiotensin II receptor antagonist used as an antihypertensive.

2. The pharmaceutical composition according to claim 1, wherein the composition is used so that the angiotensin II receptor antagonist is administered at a daily dose that is 0.1 times or less the daily dose of the angiotensin II receptor antagonist used as an antihypertensive.

3. The pharmaceutical composition according to claim 2, wherein the composition is used so that the angiotensin II receptor antagonist is administered at a daily dose that is 0.05 times or less the daily dose of the angiotensin II receptor antagonist used as an antihypertensive.

4. The pharmaceutical composition according to claim 1, wherein the angiotensin II receptor antagonist is a compound selected from the group consisting of losartan, candesartan, valsartan, telmisartan, olmesartan, irbesartan and azilsartan, or a pharmaceutically acceptable salt thereof.

5. The pharmaceutical composition according to claim 4, wherein the angiotensin II receptor antagonist is losartan.

6. The pharmaceutical composition according to any one of claims 1 to 5, wherein the organ is at least one selected from liver, kidney and heart.

7. The pharmaceutical composition according to claim 6, wherein the organ is liver and/or kidney.

8. The pharmaceutical composition according to any one of claims 1 to 5, wherein the fibrosis in the organ is fibrosis induced by a biomechanical change in the organ.

9. The pharmaceutical composition according to claim 8, wherein the biomechanical change in the organ is a change in hydrostatic pressure.
